# EUROPEAN PATENT SPECIFICATION

(11) **EP 1 535 582 B1**
(45) Date of publication and mention of the grant of the patent: **18.03.2009**
(21) Application number: 04025840.2
(22) Date of filing: 29.10.2004
(51) Int. Cl.: A61B 18/20

(54) **Light irradiation unit for diagnosing and treating skin problems**
Lichttherapiegerät zum Diagnostizieren und Behandeln von Hautproblemen
Dispositif de rayonnement lumineux pour le diagnostic et le traitement de problèmes de la peau

(30) Priority: 27.11.2003 IT bo20030717
(43) Date of publication of application: 01.06.2005
(73) Proprietor: ESPANSIONE MARKETING S.P.A., 40050 FUNO DI ARGELATO BO (IT)
(72) Inventor: Pomar, Rodolfo, 40067 Rastignano - Pianoro (IT)
(74) Representative: Modiano, Micaela Nadia

(56) References cited:
- WO-A-02/082866
- WO-A-03/043514
- WO-A-20/04096072
- GB-A- 2 369 057

## Description

The present invention relates to a light irradiation unit for diagnosing and treating skin problems and for hair removal treatments.

By subjecting the skin to light beams of various intensities and various duration, it is possible to obtain from said skin information regarding its state and characteristics, which can be used for diagnostic purposes.

Further, different combinations of light irradiation of the skin can facilitate the reduction of some kinds of dermatological disorder and improve their aesthetic appearance. For example, with treatments of this kind it is possible to eliminate or reduce the number of fine capillary vessels (telangiectasias) and to provide permanent or semipermanent hair removal on the treated surfaces.

Devices are commercially available which are provided with a lamp commonly used for flashing luminous indicators (for example sirens with flashing lamp for flying-squad vehicles), which has a higher power because it has not only a visual effect but also a heat generation effect.

The lamp is fitted inside a mirror-finished parabolic reflector, which causes the light to converge toward a specific point on the skin. All this is inserted within a handpiece provided with a button for activating the emission of light radiation.

The lamp emits radiation with a very broad wavelength range, from the ultraviolet to the infrared; accordingly, a filter that allows only the radiation of the intended wavelength to pass is installed and interposed between the lamp and the skin.

During operation, the lamp emits heat and accordingly gets hot and also heats the skin excessively.

In currently commercially available devices, the handpiece in which the lamp is mounted is cooled by means of a suction circuit; the skin of the patient is located proximate to the filter that shields the light source and is therefore struck by an intense flow of heat. The document WO 03/43514 discloses such a device, and this document discloses the preamble of claim 1.

It should be noted that the filter can be touched both intentionally (for example by inserting a finger in the front opening from which the light flashes exit) and unintentionally (for example by treating a curved skin portion and pressing the handpiece excessively against said skin, one runs the risk of contact between the skin and the surface of the filter).

Contact with the filter (which can reach high temperatures) certainly entails skin burns: therefore, this apparatus must be used with great skill and cannot be left unattended in the presence of the patient (especially after use, when the filter is still hot and burns are possible if it is handled incorrectly).

The lamp and the filter constitute an assembly by means of which it is possible to treat only some aesthetic problems: it is convenient to associate various filters with a same type of light source or to vary both the source and the filter to obtain a series of assemblies suitable for all kinds of skin and for all kinds of treatment.

Currently, the entire handpiece is replaced by adopting the one that is most suited for the type of skin and the type of treatment: this entails the purchase of a certain number of handpieces, which however are very expensive and bulky.

There are constructive solutions which use interchangeable lamp-filter assemblies: rapid replaceability allows far easier use, but over time it can entail wear of the parts that slide with respect to each other during replacement, rendering the coupling of the assembly to the handpiece scarcely stable (see, for example, WO 2004/096 072 A1).

The aim of the present invention is to obviate the cited drawbacks and meet the mentioned requirements, by providing a light irradiation unit in which contact with the filter is not possible and in which it is possible to ensure rapid replacement and anchoring of the filter and the lamp.

Within this aim, an object of the present invention is to provide a unit in which localized cooling of the handpiece is provided in order to keep at a low temperature all the parts that make contact with the operator and the patient.

Another object of the present invention is to provide a unit that is simple, relatively easy to provide in practice, safe in use, effective in operation, and has a relatively low cost.

This aim and these and other objects that will become better apparent hereinafter are achieved by a light irradiation unit, according to the invention, that has the features set forth in claim 1. Other advantageous features of the invention are set forth in the dependent claims.

Further characteristics and advantages of the present invention will become better apparent from the following detailed description of a preferred but not exclusive embodiment of a light irradiation unit, illustrated by way of non-limiting example in the accompanying drawings, wherein:

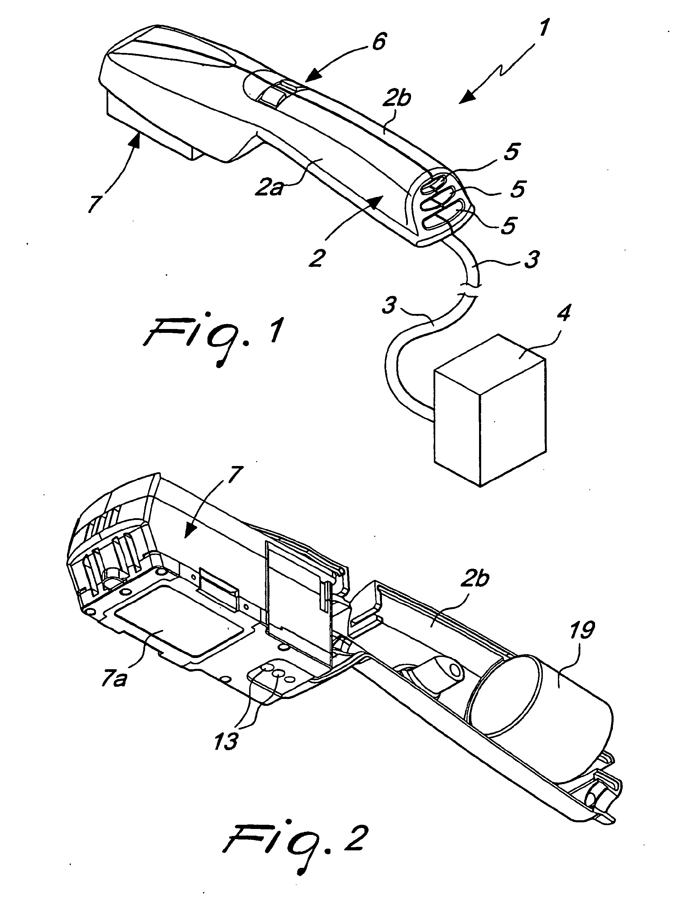

Figure 1 is a top perspective view of a handpiece of a unit according to the invention;
Figure 2 is a partially sectional bottom perspective view of a handpiece of a unit according to the invention;

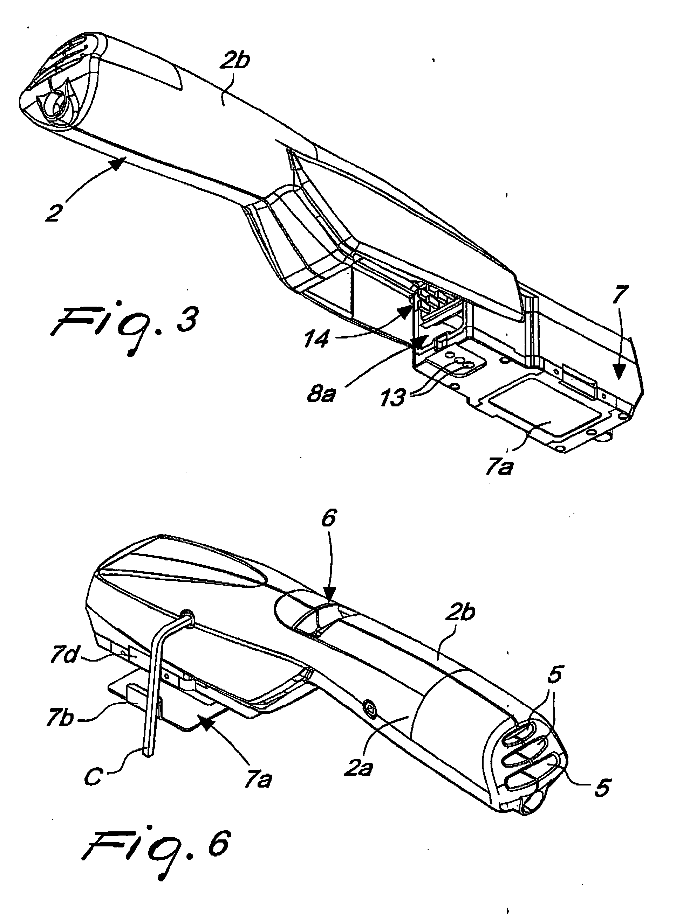

Figure 3 is a bottom perspective view of a handpiece during the extraction of a cartridge of a unit according to the invention;

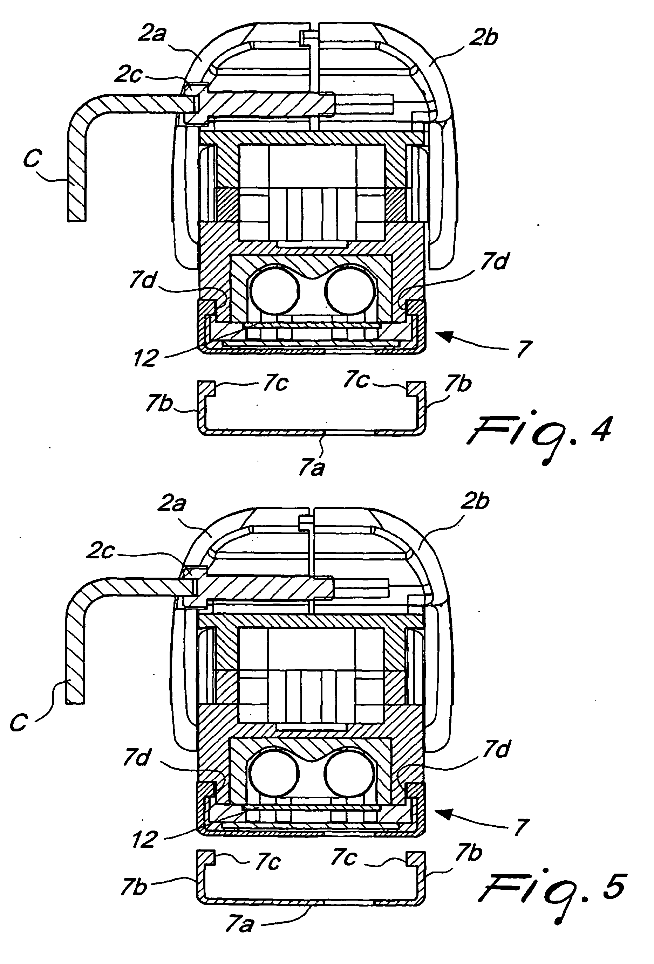

Figure 4 is a sectional front view of a handpiece of a unit according to the invention, in which the half-shells are spaced;
Figure 5 is a sectional front view of a handpiece of a unit according to the invention, in which the half-shells are closed together;
Figure 6 is a top rear perspective view of a handpiece of a unit according to the invention, in which the half-shells are spaced;

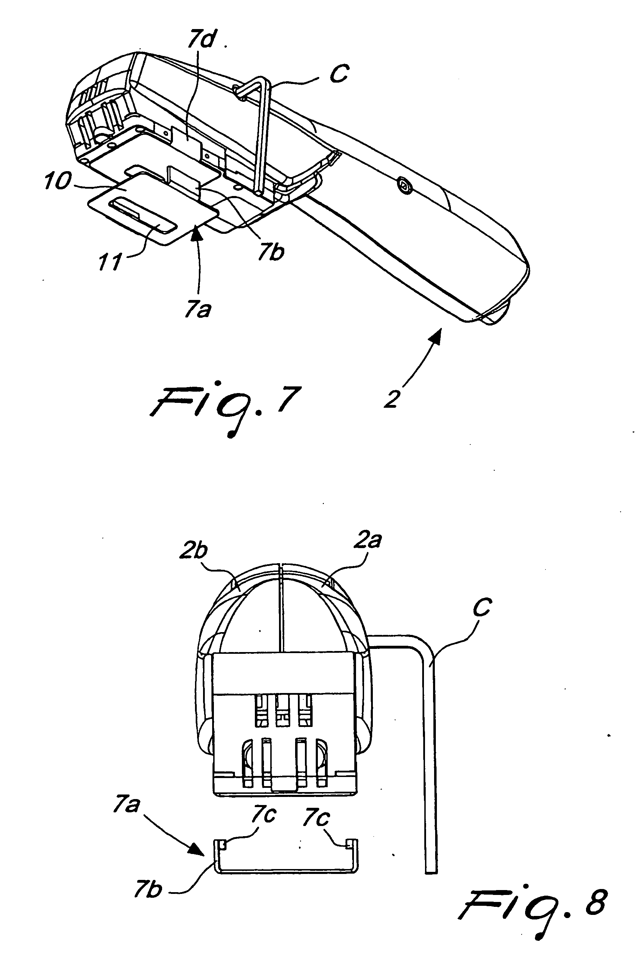

Figure 7 is a bottom front perspective view of a handpiece of a unit according to the invention, in which the half-shells are closed together;
Figure 8 is a sectional rear view of a unit according to the invention, in which the half-shells are closed together;

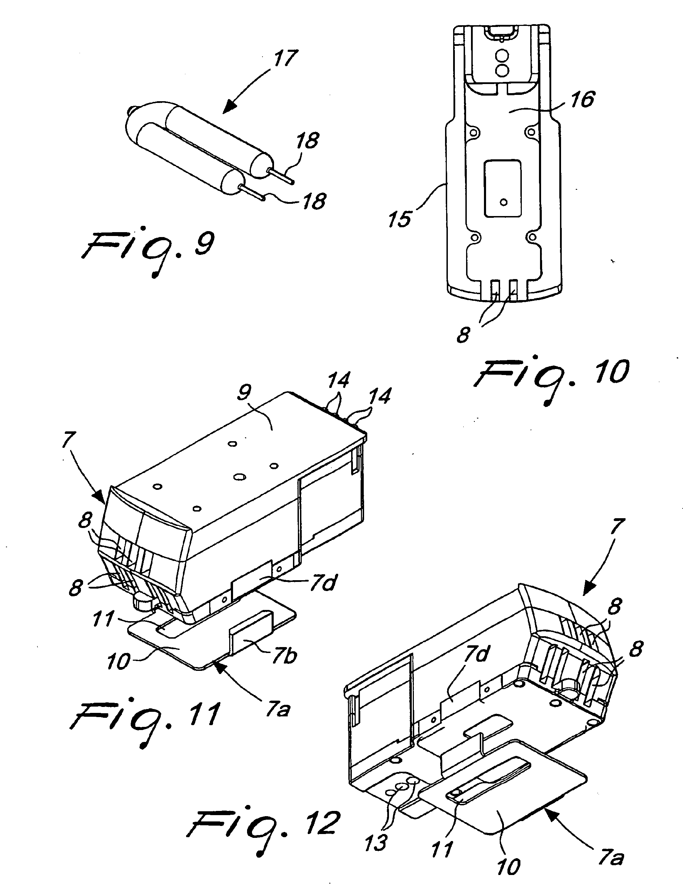

Figure 9 is a perspective view of a lamp of a unit according to the invention;
Figure 10 is a plan view of a parabolic reflector of a unit according to the invention;
Figure 11 is a front top perspective view of a cartridge of a unit according to the invention;
Figure 12 is a bottom rear perspective view of a cartridge of a unit according to the invention.

With reference to the figures, the reference numeral 1 generally designates a light irradiation unit.

The unit 1 is constituted by a handpiece 2, which is connected by means of a multicore cable 3 to a control processor 4.

The handpiece 2 has an elongated shape, with smooth and radiused surfaces; at the rear, above the hole for the insertion of the cable 3, there are through slots 5. The actuation buttons 6 are provided in an upper region, at the central portion of the handpiece 2.

Each handpiece 2 is constituted by two half-shells 2a and 2b, which are mutually coupled by means of at least one screw 2c, which passes through the half-shell 2a and engages within an appropriately provided seat of the half-shell 2b.

A cartridge 7 is accommodated in the front part of the handpiece 2 and is provided with a plurality of front openings 8 and with at least one rear slot 8a (the openings 8 and the slot 8a allow the passage of air within the cartridge 7). The cartridge 7 has the appearance of a block, with a flat bottom 9 made of a material that is not transparent to light radiation. Plates 7a are applied to the cartridge 7 and are constituted by a flat mask 10 provided with an opening 11. The cartridge 7 has, in an upper region, a transparent screen, the filter 12, which has a rectangular area with sides that measure respectively 50 and 30 mm; the filter 12 can be made of materials such as glass, quartz and sapphire but also of other plastic or ceramic materials or of other kinds of material having particular filtering actions with respect to light radiation. Beyond the filter 12, also above the cartridge 7, there is a phototype detector 13.

In the rear part of the cartridge 7 there are pins 14 for connection to the handpiece 2, which are suitable to be coupled electrically to the connection socket 14a of the handpiece 2.

The cartridge 7 is constituted by a parabolic reflector 15. Inside the parabolic reflector 15 there is a receptacle 16 for a lamp 17, which is shaped like two parallel and mutually proximate cylinders which are radiused at two ends and end, at their opposite ends, with respective electrical contacts 18. The patient can be subjected to light radiation having a wavelength comprised between 1 and 2000 nanometers.

A cooling fan 19 is installed longitudinally inside the handpiece 2 in the rear part: when the fan 19 is active, it aspirates air through the holes 5 and conveys it through a duct (delimited between the bottom 9 of the cartridge 7 and the region that surrounds the lamp 17) toward a front opening constituted by the slots 8.

The entire cartridge 7 is detachable and can be replaced with other cartridges 7 that have different filters 12, suitable for performing different therapies. For removal, it is necessary to resort to the use of a wrench C, which engages in the head of the screw 2c: by loosening the screw 2c, the two half-shells 2a and 2b are moved mutually apart and the cartridge 7 is disengaged from the clamping action, allowing its extraction; the new cartridge 7, once arranged within the half-shells 2a and 2b so that the pins 14 engage in the connection socket 14a, is fastened between the half-shells 2a and 2b, preventing its movement, by re-tightening the screw 2c with the wrench C. A plate 7a is accommodated and superimposed above the cartridge 7, is made at least partially of a material that is permeable to light radiation, and is designed to prevent direct contact of the skin of the patient with the surface of the filter 12. The surface of the filter 12, although being separate from the lamp 17, is heated considerably by said lamp, and this makes it potentially dangerous for the skin of the patient. The presence of the plate 7a, which in turn is separated from the filter 12 by means of an air gap (through which the air is conveyed forcibly by means of the fan 19), provides assurance of the patient against the risk of bums. The plate 7a is flat and is provided with two perimetric bands 7b, which are arranged at right angles thereto and are provided with end protrusions 7c for anchoring to the side walls of the cartridge 7; the cartridge 7 has, along its side walls, proximate to the open surface, from which the filter 12 can be accessed, a pair of longitudinal grooves 7d, which are suitable to accommodate stably the protrusions 7c.

The phototype detector 13 is a very important accessory, which is used to detect the type of skin being treated at a given moment. There are various kinds of skin, according to a scale defined by Fitzpatrick; this scale defines the reaction of the skin subjected to the emission of light, such as for example sunlight. This classification defines six types of skin: a first type, which always bums; a second type, which bums occasionally; a third type, which bums and occasionally tans; a fourth type, which tans and occasionally bums; a fifth type, which tans; and a sixth type, which tans and does not bum.

The phototype detector 13 allows to diagnose the patient's phototype, so as to subject the patient to the most suitable therapy.

The phototype detector 13 is arranged near the lamp 17, but can also be arranged in other parts or separately from the cartridge 7.

A temperature sensor is inserted in the cartridge 7 and is connected to the processor 4 for controlling and lighting the lamp 17; this allows to check that the lamp 17 does not exceed a certain temperature that might damage the cartridge 7 and/or bum the skin of the patient being treated. The temperature sensor can be electronic, electric, electromechanical or of any type that has these functions.

Each cartridge 7 differs according to the type of filter 12 fitted therein; in order to recognize it, the cartridge 7 is provided with an electronic recognition apparatus.

Since the filter 12 is a non-detachable part of the cartridge 7, the problem of recognizing the filter 12 is reduced to the problem of recognizing the electric circuit of the cartridge 7 on which it is installed.

The operation of the invention is as follows: once the therapy to which the patient is to be subjected is known, the type of cartridge 7 suitable for said therapy is chosen and is inserted in the handpiece 2 by sliding the bottom 9 into the appropriately provided receptacle and by making the pins 14 mate with the socket 14a; the filter detector indicates on a display the type of filter that is installed.

By pressing one of the buttons 6 arranged on the top of the handpiece 2, the phototype detector 13 is activated and identifies the characteristics of the skin of the patient, checking the reflection on the skin of the light radiation, emitted by a light source, by means of a photodiode. The result of the test is indicated on the display.

Depending on the characteristics of the skin of the patient, the operator selects the most suitable program and starts the treatment, keeping the handpiece 2 orientated so that the lamp 17 faces the portion of skin to be treated.

The temperature of the lamp 17 is monitored constantly by the temperature sensor, which sends a signal that is proportional to the temperature of the lamp 17 to the processor 4, which adjusts the duration and frequency of the flashes in succession to avoid overheating. If a temperature limit is reached, the processor 4 disables the operation of the lamp 17.

During operation, the cooling fan 19 operates and keeps the temperature of the lamp 17 low.

The simultaneous presence of cooling means that act locally (fan 19) and of a plate 7a that separates the skin of the patient from the high-temperature surfaces makes the handpiece very simple to use and protects the patient, avoiding the risk of bums.

The possibility to replace the cartridges 7 makes it easier for operators to perform the treatment; moreover, the need to fasten the inserted cartridge 7 each time gives the advantage of always achieving the correct insertion of the cartridge 7 in the handpiece 2, making sure that it cannot detach accidentally and fall (and thus certainly be damaged) or provide a poor contact between the pins 14 and the socket 14a, with consequent possible malfunction and damage of the circuits.

It has thus been shown that the invention achieves the intended aim and objects.

The invention thus conceived is susceptible of numerous modifications and variations, all of which are within the scope of the inventive concept.

For example, it is possible to block the cartridge 7 by way of other fastening means. By using a toggle lever that is articulated to a half-shell 2a and engages in a protrusion of the other half-shell 2b, it would be possible to block the cartridge 7 firmly, both due to the mutual fastening of the half-shells 2a and 2b and due to the interference between the toggle lever and the cartridge 7.

Other equally valid embodiments may provide for the presence of grub screws (or regular screws) that are rigidly coupled to the surface of the half-shells 2a and 2b and engage, by screwing, respective receptacles formed in the side walls of each cartridge 7.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A light irradiation unit for diagnosing and treating skin problems of a patient, that comprises a control processor (4) and a handpiece (2) which is connected electrically to said processor (4), the irradiation unit further comprising: a cartridge (7) that is detachably insertable in said handpiece (2) and is provided with openings (8, 8a) for the passage of air therewithin; a light source (17); and at least one filter (12), interposed between the light source (17) and a front opening of said cartridge (7) for the exit of the light; said light source (17) and at least one filter (12) being fitted in said cartridge (7), **characterized in that** said cartridge (7) is provided with a plate (7a), of a material permeable to light radiation, that is fitted thereon proximate to said filter (12) is parallel to the filter and is separated therefrom by an air gap preventing direct contact of the skin of the patient with said filter (12); and **in that** said handpiece (2) is constituted by two symmetric half-shells (2a, 2b) which are mutually coupled by way of locking means so as to constitute a chamber, said cartridge (7) being insertable in a portion of said chamber and fastenable between said half-shells (2a, 2b) by clamping action thereof upon tightening of said locking means, and **in that** said handpiece is provided with through slots at the rear (5) for enabling air passage through front and rear channel openings (8, 8a) inside said cartridge (7) and in said air gap.

2. The unit according to claim 1, **characterized in that** said cartridge (7) is shaped substantially like a parallelepiped and is provided with a plurality of electrical contacts (14) for connection to the handpiece (2).

3. The unit according to claim 1, **characterized in that** said locking means are the walls of said half-shells (2a, 2b), which can be fastened to each other by means of an appropriate wrench (C), clamping said cartridge (7) within said portion of said chamber.

4. The unit according to claim 1, **characterized in that** said locking means are threaded elements (2c), which are accommodated within through holes of said half-shells (2a, 2b) and can be turned by means of an appropriate wrench (C), fastening said cartridge (7) within said portion of said chamber.

5. The unit according to claim 1, **characterized in that** said locking means are constituted by at least one toggle lever, which is articulated on one of said half-shells (2a), engages in a protrusion of the other half-shell (2b), and is suitable to hinder the exit of said cartridge (7) from said portion of said chamber.

6. The unit according to claim 1, **characterized in that** said plate (7a) that is permeable to light radiation has two perimetric bands (7b), which are arranged at right angles thereto and are provided with end protrusions (7c) for anchoring to the side walls of said cartridge (7).

7. The unit according to claims 1 and 6, **characterized in that** said cartridge (7) has, along its side walls, proximate to the open surface, from which said filter (12) can be accessed, two longitudinal grooves (7c), which are suitable to accommodate stably said protrusions (7b) of said plate (7a).

8. The unit according to claim 1, **characterized in that** said plate (7a) is made of glassy material.

9. The unit according to claim 1 and as an alternative to claim 8, **characterized in that** said plate (7a) is made of crystalline material.

10. The unit according to claim 1, **characterized in that** said plate (7a) is made of polymeric material.

11. The unit according to one or more of the preceding claims, **characterized in that** the light radiation emitted by said light source (17) has wavelengths comprised between 1 and 2000 nanometers.

12. The unit according to one or more of the preceding claims, **characterized in that** inside said handpiece (2) and said cartridge (7), all the components are provided mounted so that they are mutually spared in order to facilitate convection for cooling.

13. The unit according to one or more of the preceding claims, **characterized in that** said handpiece (2) comprises a small electric motor, a fan (19) driven by said motor and which is installed upstream of a duct arranged inside the handpiece (2), which is connected to the outside at the front and at the rear of said handpiece (2); said motor, said fan (19) and said duct being suitable to cool said light source (17) installed in said cartridge (7).

14. The unit according to one or more of the preceding claims, **characterized in that** said handpiece (2) comprises a hydraulic circuit, which is constituted by a continuous channel that is distributed along the handpiece and is connected, by means of a delivery tube and a return tube, to a refrigeration unit.

15. The unit according to claim 1, **characterized in that** said light source (17) is suitable to affect a rectangular skin treatment area which has an area of approximately 5 and 3 centimeters.

16. The unit according to claim 1, **characterized in that** said cartridge (7) containing said light source (17) is provided with masks (10) for reducing the treatment area, which can be superimposed on said plate (7a).

17. The unit according to claim 16, **characterized in that** said masks (10) are constituted by plates made of non-transparent material, which are provided with small openings (11) and are installed on respective sliders (7d) along the lateral surfaces of the cartridge (7) that contains the light source (17), said masks (10) being able to perform a translational motion and a rotation from a configuration in which they are parallel and proximate to said lateral surfaces to a configuration in which they are interposed between said light source (17) and the skin, reducing the illuminated area.

## Patentansprüche

1. Lichtbestrahlungseinheit zum Diagnostizieren und Behandeln von Hautproblemen eines Patienten, die einen Steuerprozessor (4) und ein Handteil (2), das mit dem Prozessor (4) elektrisch verbunden ist, umfasst, wobei die Bestrahlungseinheit ferner umfasst: eine Kassette (7), die in das Handteil (2) lösbar einsteckbar ist und mit Öffnungen (8, 8a) für den Durchgang von Luft durch sie hindurch versehen ist; eine Lichtquelle (17); und wenigstens ein Filter (12), das zwischen die Lichtquelle (17) und eine vordere Öffnung der Kassette (7) für den Austritt von Licht eingefügt ist; wobei die Lichtquelle (17) und wenigstens ein Filter (12) in der Kassette (7) angebracht sind, **dadurch gekennzeichnet, dass** die Kassette (7) mit einer Platte (7a) aus einem für Lichtstrahlung durchlässigen Material versehen ist, die daran in der Nähe des Filters (12) parallel zu dem Filter angebracht ist und hiervon durch einen Luftspalt, der einen direkten Kontakt der Haut des Patienten mit dem Filter (12) verhindert, getrennt ist; **dass** das Handteil (2) aus zwei symmetrischen Halbschalen (2a, 2b) gebildet ist, die durch Verriegelungsmittel miteinander gekoppelt sind, um eine Kammer zu bilden, wobei die Kassette (7) in einen Abschnitt der Kammer einführbar und zwischen den Halbschalen (2a, 2b) durch Klemmwirkung beim Anziehen der Verriegelungsmittel befestigbar ist, und **dass** das Handteil am hinteren Ende (5) mit Durchgangsschlitzen versehen ist, um den Luftdurchgang durch eine vordere und eine hintere Kanalöffnung (8, 8a) in der Kassette (7) und in dem Luftspalt zu ermöglichen.

2. Einheit nach Anspruch 1, **dadurch gekennzeichnet, dass** die Kassette (7) im Wesentlichen wie ein Parallelepiped geformt ist und mit mehreren elektrischen Kontakten (14) für die Verbindung mit dem Handteil (2) versehen ist.

3. Einheit nach Anspruch 1, **dadurch gekennzeichnet, dass** die Verriegelungsmittel Wände der Halbschalen (2a, 2b) sind, die aneinander mittels eines geeigneten Schlüssels (C) befestigt werden können und die Kassette (7) in dem Abschnitt der Kammer festklemmen.

4. Einheit nach Anspruch 1, **dadurch gekennzeichnet, dass** die Verriegelungsmittel Gewindeelemente (2c) sind, die in Durchgangslöcher der Halbschalen (2a, 2b) aufgenommen sind und mittels eines geeigneten Schlüssels (C) gedreht werden können, wodurch die Kassette (7) in dem Abschnitt der Kammer befestigt wird.

5. Einheit nach Anspruch 1, **dadurch gekennzeichnet, dass** die Verriegelungsmittel durch wenigstens einen Kniehebel gebildet sind, der an einer der Halbschalen (2a) angelenkt ist, in einem Vorsprung der anderen Halbschale (2b) in Eingriff ist und eine Herausbewegung der Kassette (7) aus dem Abschnitt der Kammer verhindern kann.

6. Einheit nach Anspruch 1, **dadurch gekennzeichnet, dass** die für Lichtstrahlung durchlässige Platte (7a) zwei symmetrische Umfangsbänder (7b) besitzt, die rechtwinklig hierzu angeordnet sind und mit Endvorsprüngen (7c) versehen sind, um an den Seitenwänden der Kassette (7) verankert zu werden.

7. Einheit nach den Ansprüchen 1 und 6, **dadurch gekennzeichnet, dass** die Kassette (7) längs ihrer Seitenwände in der Nähe der offenen Oberfläche, von der aus auf das Filter (12) zugegriffen werden kann, zwei longitudinale Nuten (7c) besitzt, die die Vorsprünge (7b) der Platte (7a) stabil aufnehmen können.

8. Einheit nach Anspruch 1, **dadurch gekennzeichnet, dass** die Platte (7a) aus einem glasartigen Material hergestellt ist.

9. Einheit nach Anspruch 1 und alternativ nach Anspruch 8, **dadurch gekennzeichnet, dass** die Platte (7a) aus einem kristallinen Material hergestellt ist.

10. Einheit nach Anspruch 1, **dadurch gekennzeichnet, dass** die Platte (7a) aus einem Polymermaterial hergestellt ist.

11. Einheit nach einem oder mehreren der vorhergehenden Ansprüche, **dadurch gekennzeichnet, dass** die durch die Lichtquelle (17) ausgesendete Lichtstrahlung Wellenlängen im Bereich von 1 bis 2000 Nanometer hat.

12. Einheit nach einem oder mehreren der vorhergehenden Ansprüche, **dadurch gekennzeichnet, dass** in dem Handteil (2) und in der Kassette (7) alle Komponenten so montiert vorgesehen sind, dass sie in gegenseitigem Abstand sind, um eine Konvektionskühlung zu erleichtern.

13. Einheit nach einem oder mehreren der vorhergehenden Ansprüche, **dadurch gekennzeichnet, dass** das Handteil (2) einen kleinen Elektromotor, einen durch den Motor angetriebenen Lüfter (19), der stromaufseitig von einem in dem Handteil (2) angeordneten Rohrstutzen installiert ist, der seinerseits am vorderen Ende und am hinteren Ende des Handteils (2) mit der äußeren Umgebung verbunden ist, umfasst; wobei der Motor, der Lüfter (19) und der Rohrstutzen die in der Kassette (7) installierte Lichtquelle (17) kühlen können.

14. Einheit nach einem oder mehreren der vorhergehenden Ansprüche, **dadurch gekennzeichnet, dass** das Handteil (2) einen Hydraulikkreis umfasst, der durch einen ununterbrochenen Kanal gebildet ist, der längs des Handteils verteilt ist und über ein Zufuhrrohr und ein Rücklaufrohr mit einer Kühlungseinheit verbunden ist.

15. Einheit nach Anspruch 1, **dadurch gekennzeichnet, dass** die Lichtquelle (17) einen rechtwinkligen Hautbehandlungsbereich, der eine Fläche von etwa 5 mal 3 cm hat, beaufschlagen kann,

16. Einheit nach Anspruch 1, **dadurch gekennzeichnet, dass** die Kassette (7), die die Lichtquelle (17) enthält, mit Masken (10) zum Verkleinern des Behandlungsbereichs, die der Platte (7a) überlagert werden können, versehen ist.

17. Einheit nach Anspruch 16, **dadurch gekennzeichnet, dass** die Masken (10) durch Platten aus einem lichtundurchlässigen Material gebildet sind, die mit kleinen Öffnungen (11) versehen sind und an entsprechenden Gleitern (7d) längs der seitlichen Oberflächen der Kassette (7), die die Lichtquelle (17) enthält, installiert sind, wobei die Masken (10) eine translatorische Bewegung und eine Drehung aus einer Konfiguration, in der sie zu den seitlichen Oberflächen parallel sind und sich in deren Nähe befinden, in eine Konfiguration, in der sie zwischen die Lichtquelle (17) und die Haut eingefügt sind und den beleuchteten Bereich verringern, ausführen können.

## Revendications

1. Unité de rayonnement lumineux pour le diagnostic et le traitement de problèmes de la peau d'un patient, qui comprend un processeur de commande (4) et une pièce à main (2), qui est connectée électriquement audit processeur (4), l'unité de rayonnement comprenant en outre : une cartouche (7) qui peut être insérée de façon détachable dans ladite pièce à main (2) et est pourvue d'ouvertures (8, 8a) pour le passage d'air à l'intérieur de celles-ci ; une source lumineuse (17) ; et d'au moins un filtre (12), interposé entre la source lumineuse (17) et une ouverture avant de ladite cartouche (7) pour la sortie de la lumière : ladite source (17) et au moins un filtre (12) étant installés dans ladite cartouche (7), **caractérisée en ce que** ladite cartouche (7) est pourvue d'une plaque (7a), d'un matériau perméable au rayonnement lumineux, qui est installée sur celle-ci à proximité dudit filtre (12) parallèlement au filtre et est séparée de celle-ci par un espace d'air empêchant le contact direct de la peau du patient avec ledit filtre (12) ; et **en ce que** ladite pièce à main (2) est constituée par deux demi-coques symétriques (2a, 2b), qui sont couplées mutuellement par l'intermédiaire de moyens de verrouillage afin de constituer une chambre, ladite cartouche (7) pouvant être insérée dans une partie de ladite chambre et pouvant être fixée entre lesdites demi-coques (2a, 2b) par action de serrage de celles-ci lors du serrage desdits moyens de verrouillage et **en ce que** ladite pièce à main est pourvue de fentes débouchantes à l'arrière (5) pour permettre le passage d'air à travers les ouvertures de canal avant et arrière (8, 8a) à l'intérieur de ladite cartouche (7) et dans ledit espace d'air.

2. Unité selon la revendication 1, **caractérisée en ce que** ladite cartouche (7) est formée sensiblement comme un parallélépipède et est pourvue d'une pluralité de contacts électriques (14) pour la connexion à la pièce à main (2).

3. Unité selon la revendication 1, **caractérisée en ce que** lesdits moyens de verrouillage sont les parois desdites demi-coques (2a, 2b), qui peuvent être fixées l'une à l'autre au moyen d'une clef appropriée (C), serrant ladite cartouche (7) à l'intérieur de ladite partie de ladite chambre.

4. Unité selon la revendication 1, **caractérisée en ce que** lesdits moyens de verrouillage sont des éléments filetés (2c), qui sont logés à l'intérieur de trous débouchants desdites demi-coques (2a, 2b) et peuvent être tournés au moyen d'une clef appropriée (C), fixant ladite cartouche (7) à l'intérieur de ladite partie de ladite chambre.

5. Unité selon la revendication 1, **caractérisée en ce que** lesdits moyens de verrouillage sont constitués par au moins un levier articulé, qui est articulé sur une desdites demi-coques (2a), s'engage dans une protubérance de l'autre demi-coque (2b), et est approprié pour empêcher la sortie de ladite cartouche (7) à partir de ladite partie de ladite chambre.

6. Unité selon la revendication 1, **caractérisée en ce que** ladite plaque (7a) qui est perméable au rayonnement lumineux comporte deux bandes périmétriques (7b), qui sont agencées à angle droit par rapport à celle-ci et sont pourvues de protubérances d'extrémité (7c) pour s'ancrer aux parois latérales de ladite cartouche (7).

7. Unité selon les revendications 1 et 6, **caractérisée en ce que** ladite cartouche (7) comporte, le long de ses parois latérales, à proximité de la surface ouverte, à partir desquelles il est possible d'accéder audit filtre (12), deux rainures longitudinales (7c), qui sont appropriées pour contenir de façon stable lesdites protubérances (7b) de ladite plaque (7a).

8. Unité selon la revendication 1, **caractérisée en ce que** ladite plaque (7) est faite de matériau vitreux.

9. Unité selon la revendication 1 et en variante selon la revendication 8, **caractérisée en ce que** ladite plaque (7) est faite de matériau cristallin.

10. Unité selon la revendication 1, **caractérisée en ce que** ladite plaque (7) est faite de matériau polymère.

11. Unité selon une ou plusieurs des revendications précédentes, **caractérisée en ce que** le rayonnement lumineux émis par ladite source lumineuse (17) comporte des longueurs d'onde comprises entre 1 et 2000 nanomètres.

12. Unité selon une ou plusieurs des revendications précédentes, **caractérisée en ce qu'**à l'intérieur de ladite pièce à main (2) et ladite cartouche (7), tous les composants sont montés de sorte qu'ils soient mutuellement espacés afin de faciliter la convection pour le refroidissement.

13. Unité selon une ou plusieurs des revendications précédentes, **caractérisée en ce que** ladite pièce à main (2) comprend un moteur électrique de petite taille qui entraîne un ventilateur (19) qui est installé en amont d'une conduite agencée à l'intérieur de la pièce à main (2), qui est connectée à l'extérieur à l'avant et à l'arrière de ladite pièce à main (2) ; ledit moteur, ledit ventilateur (19) et ladite conduite étant appropriés pour refroidir ladite source lumineuse (17) installée dans ladite cartouche (7).

14. Unité selon une ou plusieurs des revendications précédentes, **caractérisée en ce que** ladite pièce à main (2) comprend un circuit hydraulique, qui est constitué par un canal continu qui est distribué le long de la pièce à main et est connecté, au moyen d'un tube de distribution et d'un tube de retour, à une unité de réfrigération.

15. Unité selon la revendication 1, **caractérisée en ce que** ladite source lumineuse (17) affecte une zone de traitement cutanée rectangulaire qui comporte une superficie d'approximativement 5 et 3 centimètres.

16. Unité selon la revendication 1, **caractérisée en ce que** ladite cartouche (7) contenant ladite source lumineuse (17) est pourvue de masques (10) pour réduire la zone de traitement, qui peuvent être superposés sur ladite plaque (7a).

17. Unité selon la revendication 16, **caractérisée en ce que** lesdits masques (10) sont des plaques faites de matériau non transparent, qui sont pourvues d'ouvertures (11) de petite taille et sont installées sur des coulisseaux (7d) respectifs le long des surfaces latérales de la cartouche (7) qui contient la source lumineuse (17), lesdits masques (10) étant capables de réaliser un mouvement de translation et une rotation à partir d'une configuration dans laquelle ils sont parallèles et à proximité desdites surfaces latérales jusqu'à une configuration dans laquelle ils sont interposés entre ladite source lumineuse (17) et la peau, réduisant la superficie illuminée.
